(19) Europäisches Patentamt
European Patent Office
Office européen des brevets

(11) Publication number: **0 175 171**
**A2**

(12) **EUROPEAN PATENT APPLICATION**

(21) Application number: **85110622.9**

(22) Date of filing: **23.08.85**

(51) Int. Cl.[4]: **A 61 K 31/19**

(30) Priority: **31.08.84 JP 182384/84**

(43) Date of publication of application: **26.03.86**
**Bulletin 86/13**

(84) Designated Contracting States: **BE CH DE FR GB IT LI NL SE**

(71) Applicant: **Eisai Co., Ltd., 6-10, Koishikawa 4-chome Bunkyo-ku, Tokyo 112 (JP)**

(72) Inventor: **Seto, Toshio, 3420-55, Ushikumachi Ushiku, Inashiki-gun Ibaraki (JP)**
Inventor: **Suzuki, Yoshikazu, 13-1, Jingumae 5-chome, Shibuya-ku Tokyo (JP)**

(74) Representative: **Hansen, Bernd, Dr.rer.nat. et al, Hoffmann, Eitle & Partner Patentanwälte Arabellastrasse 4, D-8000 München 81 (DE)**

(54) **Use of 3,7,11,15-tetramethyl-2,4,6,10,14-hexadecapentaenoic acid for enhancing the effect of anti-tumour agents.**

(57) 3,7,11,15-tetramethyl-2,4,6,10,14-hexadecapentaenoic acid or a salt thereof can enhance the anti-tumor effect of an anti-tumor agent.

EP 0 175 171 A2

ACTORUM AG

0175171
42 520 m/kü

## Pharmaceutical Agent to Enhance the Anti-Tumor Effect

The invention relates to a pharmaceutical agent to enhance the anti-tumor effect. In particular, the agent comprises 3,7,11,15-tetramethyl-2,4,6,10,14-hexadecapentaenoic acid of the formula (1) or a salt thereof.

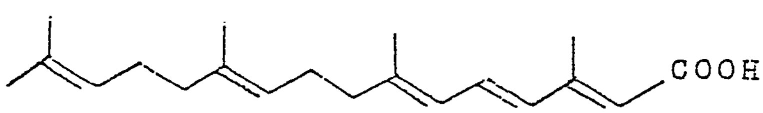 (I)

In the field of chemotherapy of cancer where the development of novel carcinostatic drugs is becoming more and more difficult all the world over, it is a very important problem to try to enhance the effect of known carcinostatic drugs.

The inventors of the present invention have long studied on a carcinostatic synergist and have found that 3,7,11,15-tetramethyl-2,4,6,10,14-hexadecapentaenoic acid or its salt (I) is remarkably effective in enhancing a carcinostatic effect. The present invention has been accomplished based on this finding.

The compound (I) of the present invention has also been found to be effective as an antineoplastic drug or a medicine for dermatopathy accompanied by carnification and applications for patent with respect to this compound have been filed by the same applicant as the one of the present invention [Japanese Patent Application Nos. 44558/1980 and 104420/1980 (Japanese Patent Laid-Open Nos. 140946/1981 and 31615/1982)].

Thereafter, the inventors of the present invention have further studied on the effect of this compound in more detail and have found that the compound exhibits a carcinostatic effect-enhancing effect, so that it is used jointly with other known carcinostatic drugs to enhance the carcinostatic effect thereof remarkably.

The invention provides a method for enhancing the anti-tumor effect, which comprises administering a therapeutically effective amount of 3,7,11,15-tetramethyl-2,4,6,10,14-hexadecapentaenoic acid or a salt thereof in combination with an anti-tumor agent to a patient suffering from tumor. It further provides a pharmaceutical composition which comprises (A) 3,7,11,15-tetramethyl-2,4,6,10,14-hexadecapentaenoic acid or a salt thereof and (B) an anti-tumor agent. Alternatively, the invention may be defined as a pharmaceutical use of 3,7,11,15-tetramethyl-2,4,6,10,14-hexadecapentaenoic acid or a salt thereof for enhancing the anti-tumor effect. In other words, the invention concerns a pharmaceutical use of 3,7,11,15-tetramethyl-2,4,6,10,14-hexadecapentaenoic acid or a salt thereof for preparing a pharmaceutical composition to enhance the anti-tumor effect.

The pharmaceutical agent to enhance the anti-tumor effect or the carcinostatic drug to use in the invention may be any one including those which will be developed in the future. Examples of the commercially available carcinostatic drugs which are used clinically and exhibit a good result include fluorouracil(5-FU), mitomycin(MMC), doxorubicin hydrochloride(ADM), bleomycin hydrochloride(BLM), cytarabine(Ara-C), pepleomycin sulfate(PEP), minustine hydrochloride(ACNU), carboquone(CQ), cisplatin(CDDP), Thio TEPA, vincristine sulfate(VCR), methotrexate (MTX) and cyclophosphamide(CPM).

The compound (I) of the present invention is 3,7,11,15-tetramethyl-2,4,6,10,14-hexadecapentaenoic acid represented by the formula:

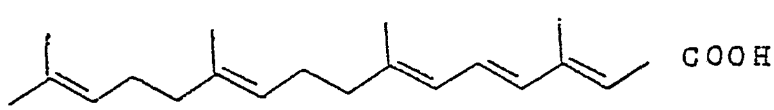

or its salt. Examples of the salt include sodium and potassium salts.

The compound (I) of the present invention itself has a carcinostatic effect as described above. Further, when it is used together with other carcinostatic drugs, a carcinostatic effect which is remarkably enhanced by the simultaneous use of the compound (I) and other drugs can be obtained, though the mechanism is not clear.

The compound (I) of the present invention is of a quite new type structurally as a carcinostatic drug or a carcinostatic synergist. The compound (I) exhibits a remarkably low toxicity, so that it is highly valuable clinically owing to its high safety.

For reference, processes for preparing the compound of the present invention will be described.

Method A

(i) A compound represented by the general formula (II):

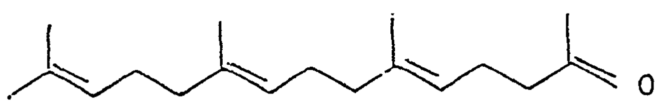

(II)

is reacted with a Wittig reagent derived from a compound represented by the general formula (III):

$$X - CH_2 - CO_2R_1 \qquad (III)$$

wherein X stands for a halogen atom and $R_1$ stands for a lower alkyl group,

to prepare a compound represented by the general formula (IV):

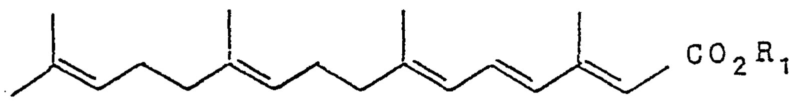 (IV)

wherein $R_1$ is as defined above.

(ii) The obtained compound represented by the general formula (IV) is hydrolyzed in the presence of a base to obtain the compound (I).

Examples of the Wittig reagent derived from a compound represented by the general formula (III) to be used in the above step (i) include phosphorus compounds obtained by reacting the compound represented by the general formula (III) with triphenylphosphine, phenyldialkoxyphosphine, trialkyl phosphite or the like. The preparation of the reagent and the Wittig reaction with this reagent can be carried out by an ordinary method, such as Wadworth's method [J. Am. Chem. Soc., 83, 1733 (1961)], Greenwald's method [J. Org. Chem., 28, 1128 (1963)] or Horner's method [Ber. 95, 581 (1962)].

The hydrolysis in the above step (ii) can be carried out by using a base which is generally used

in the hydrolysis of a carboxylate, such as sodium hydroxide or potassium hydrooxide.

Method B

(i) A compound represented by the general formula (V):

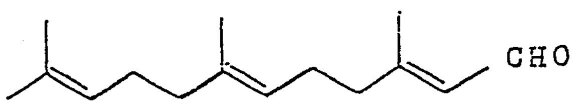

(V)

is reacted with a Wittig reagent derived from a compound represented by the general formula (VI):

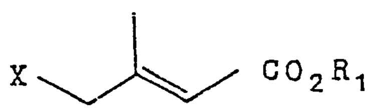

(VI)

wherein X stands for a halogen atom and $R_1$ stands for a lower alkyl group,

to prepare a compound represented by the general formula (IV).

(ii) The obtained compound represented by the general formula (IV) is hydrolyzed in the presence of a base to obtain the compound (I).

These steps (i) and (ii) can be carried out in a similar manner as described in Method A.

Method C

(i) A compound represented by the general formula (VII):

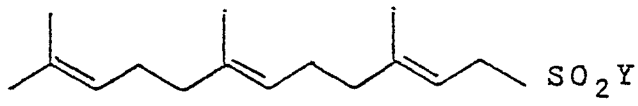

(VII)

wherein Y stands for a lower alkyl or aryl group, is reacted with a compound represented by the general formula (VI) to prepare a compound represented by the general formula (VIII):

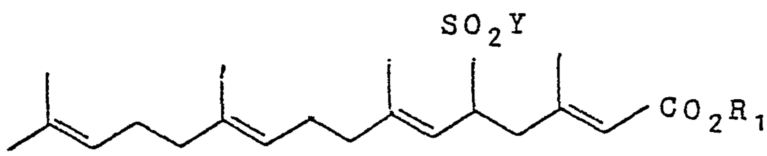

(VIII)

wherein $R_1$ and Y are as defined above.

(ii) The obtained compound represented by the general formula (VIII) is desulfinated and hydrolyzed in the presence of a base to obtain the compound (I).

The above step (i) is carried out in the presence of a base. Examples of the base include n-butyllithium and phenyllithium. Examples of the solvent to be used in the step (i) include tetrahydrofuran, diethyl ether and 1,2-dimethoxyethane. The reaction is generally carried out at a room temperature or below.

Examples of the substituent X in the general formulae (III), (IV), (VI), (VII) and (VIII) include halogen atoms such as chlorine, bromine or iodine, while those of the substituent $R_1$ include lower alkyl groups such as methyl, ethyl and propyl groups.

Examples of the substituent Y include lower alkyl groups such as methyl, ethyl and propyl groups and aryl groups such as phenyl and p-tolyl groups.

The results of pharmacological tests with the compound of the present invention will be described in the following Experimental Examples in order to illustrate the effect of the present invention in more detail.

Experimental Example 1

Effect of the simultaneous use of a known carcinostatic agent and the compound (I) (Enhancement of cytocidal effect)

(1) Experimental material and procedure

① Cell: Epithelical cell from human uterocervical cancer, HeLa.S3 cell

② Cultivation: The cell was cultivated using a culture fluid obtained by adding 10 parts of fetal calf serum to 90 parts of Eagles Minimum Essential Medium in an incuvator having a carbon dioxide concentration controlled to 5% at 37°C.

③ Drug: The compound of the present invention was added as a solution in dimethyl sulfoxide(DMSO) of a final DMSO concentration of 0.1% to the culture fluid. The clinical injections as shown in Table 1 were added to the culture fluid as a

known carcinostatic drug.

Table 1

| Abbreviation | Main component | Trade mark |
|---|---|---|
| 5FU | fluorouracil | 5-FU Kyowa |
| MMC | mitomycin C | Mitomycin Kyowa |
| ADM | doxorubicin hydro-chloride | Adriacin Injection |
| BLM | bleomycin hydro-chloride | Bleo |
| Ara-C | cytarabine | Cylocide Injection |
| PEP | pepleomycin sulfate | Pepleo Injection |
| ACNU | nimustine hydro-chloride | Nidran Injection |
| CQ | carboquone | Exiquinon for Injection |
| CDDP | cisplatin | Randa Injection |
| Thio.TEPA | thio.tepa | Tespamin Injection |
| VCR | vincristine sulfate | Oncovin |
| CPM | cyclophosphamide | Endoxan for Injection |

(4) Experimental procedure: 150 epithelical cells from human uterocervical cancer (HeLaS3 cell) were planted in each plastic dish of a diameter of 60 mm. After 4 hours, the compound of the present invention and each carcinostatic drug were simultaneously added to the dish to give a final concentration with respect to the compound of the

present invention of 0, $10^{-6}$, $10^{-5}$ or $3.2X\ 10^{-5}$ mol(M) and various final concentration with respect to the drugs. After the cultivation for 7 days, the cells were fixed with methanol and subjected to Giemsa's staining, followed by calculation of the number of colonies containing at least 20 cells under a stereomicroscope. The concentration (ng/mℓ) of the carcinostatic drug required to decrease the ratio of the number of colonies of a treated group to that of an untreated group (control) to 50% were determined and are shown as "$IC_{50}$".

With respect to CPM, after the cells had been cultivated in the presence of the compound of the present invention for 3 days, they were added in the presence of an S9 mix obtained by adding coenzyme or the like to an S9 fraction of the rat liver, followed by determination of $IC_{50}$ in a similar manner as above.

The results are shown in Table 2.

Table 2

| carcino-static drug | | IC$_{50}$ (ng/ml) | | | |
|---|---|---|---|---|---|
| | | 0[a)] | $10^{-6}$[b)] (a/b) | $10^{-5}$[c)] (a/c) | $3.2\times10^{-5}$[d)] (a/d) |
| 5-FU | 1) | 648 | 375 (1.73*) | 329 (1.97*) | 267 (2.43*) |
| | 2) | 648 | 388 (1.67*) | 369 (1.76*) | 347 (1.87*) |
| Ara-C | 1) | 10.9 | 11.9 (0.92) | 11.4 (0.96) | 6.25 (1.74*) |
| | 2) | 10.9 | 12.0 (0.91) | 12.4 (0.88) | 11.6 (0.94*) |
| MMC | 1) | 1.50 | 1.05 (1.43) | 0.74 (2.03*) | 0.40 (3.75*) |
| | 2) | 1.50 | 1.06 (1.42) | 0.82 (1.69*) | 0.84 (1.79*) |
| ADM | 1) | 3.79 | 3.10 (1.22) | 2.43 (1.56*) | 1.88 (2.02*) |
| | 2) | 3.79 | 3.51 (1.08) | 4.15 (0.91) | 3.14 (1.21) |
| PEP | 1) | 86.0 | 75.2 (1.14) | 4.51 (1.91*) | 14.3 (6.01*) |
| | 2) | 86.0 | 73.9 (1.16) | 53.1 (1.62*) | 36.2 (2.38*) |
| BLM | 1) | 450 | 430 (1.05) | 217 (2.07*) | 61.1 (7.36*) |
| | 2) | 450 | 360 (1.25) | 490 (0.92) | 120 (3.75*) |
| ACNU | 1) | 135 | 143 (0.94) | 146 (0.92) | 102 (1.32) |
| | 2) | 135 | 147 (0.92) | 150 (0.90) | 136 (0.99) |
| CQ | 1) | 0.339 | 0.273 (1.24) | 0.242 (1.40) | 0.076 (4.46*) |
| | 2) | 0.339 | 0.253 (1.34) | 0.275 (1.23) | 0.131 (2.59*) |
| CDDP | 1) | 45.8 | 34.0 (1.35) | 37.8 (1.21) | 37.9 (1.21) |
| | 2) | 45.8 | 37.8 (1.21) | 42.9 (1.07) | 59.2 (0.77) |
| Thio-TEPA | 1) | 311 | 274 (1.14) | 299 (1.04) | 274 (1.14) |
| | 2) | 311 | 291 (1.07) | 319 (0.97) | 244 (1.27) |
| VCR | 1) | 5.50 | 7.44 (0.74) | 6.38 (0.86) | 2.63 (2.09*) |
| | 2) | 5.50 | 7.45 (0.74) | 7.70 (0.71) | 5.65 (0.97) |
| CPM | 1) | 4210 | 3358 (1.25) | 1326 (3.17*) | 1225 (3.44*) |
| | 2) | 4210 | 3165 (1.33) | 1974 (2.13*) | 2304 (1.83*) |

The figures in Table 2 represent the $IC_{50}$ values of the carcinostatic drugs. The values in parentheses represent the ratios of $IC_{50}$ at a concentration of the compound of the present invention of $10^{-6}$M, $10^{-5}$M or $3.2 \times 10^{-5}$M to that at a concentration of the compound of OM. That is to say, these values show the degree of the enhancement of the cytocidal effect of the carcinostatic drugs which was attained by the compound of the present invention.

The figure in the upper column 1) of each carcinostatic drug represents the $IC_{50}$ value based on the case where the concentrations of both the compound of the present invention and each drug are 0.

The figure in the lower column 2) of each carcinostatic drug represents the $IC_{50}$ value based on the case where the concentration of the compound of the present invention is $10^{-6}$, $10^{-5}$ or $3.2 \times 10^{-5}$M, respectively, and that of the drug is 0. The cases where the degree of the enhancement of the cytocidal effect is 1.50 or above are marked by "*".

It is clear from the figures of Table 2 that the compound of the present invention synergisticly enhances the cytocidal effect of a carcinostatic drug. The effect is particularly remarkable in the case of 5FU, MMC, PEP, BLM, CQ or CPM.

Experimental Example 2

Toxicological test

The compound of the present invention, 3,7,11,15-tetramethyl-2,4,6,10,14-hexadecapentaenoic acid, was administered orally to each group consisting of six female ICR mice for 14 days at a dosage of 40, 200 or 400 mg/kg/day, respectively, to observe changes in the weight, the mortality or the like.

No mice died and an adverse reaction such as decrease in weight or cyanosis was not observed.

It should be understood from the above Experimental Examples 1 and 2 that the compound of the present invention is remarkably effective in enhancing a carcinostatic effect and that it is used together with a carcinostatic drug to remarkably enhance the carcinostatic effect. Additionally, it is clear from the toxicological test that the compound of the present invention is highly safe, so that the present invention is highly valuable in this sense, because a carcinostatic drug must be administered for a long time from the nature of the disease.

When the compound of the present invention is used together with other carcinostatic drug as a carcinostatic synergist, it may be administered in the form of a previously prepared compound preparation containing

the compound and the carcinostatic drug, for example, in the form of injection, powder, granular, tablet or capsule. Alternatively, the compound and the carcinostatic drug may be administered simultaneously and separately to patients.

The carcinostatic drug to be used together with the compound of the present invention may be any one in a very wide range including existing ones and ones which will be developed in the future. Representative examples of the drugs which exhibit good results include those based on vinca alkaloid such as vincristine, vinblastine, vindesine and VF16; adriamycin compounds such as doxorubicin hydrochloride (adriamycin) and daunomycin; 5-FU compounds, mitomycin C, bleomycin hydrochloride, cytarabine, pepleomycin sulfate, nimustine hydrochloride, carboquone, cisplatin and Thio tepa.

When the compound of the present invention is used as a carcinostatic synergist, the amount to be administered is not particularly limited and is varied depending upon the kind of a carcinostatic drug, the kind of cancer, the condition of a patient or the like. Generally, the compound is administered orally or nonorally at a dosage of about 10 to 2000 mg, preferably 5 to 500 mg per adult a day. The compound

can be administered in the form of injection, powder, fine granule, granule, tablet, capsule or the like. The preparation is carried out using an ordinary carrier in a conventional manner.

More particularly, when a solid preparation for oral administration is manufactured, a principal agent is mixed with a vehicle, if necessary, together with binder, disintegrating agent, lubricant, coloring agent or corrigent, and treated according to an ordinary method to be formed into tablet, coated tablet, granule, capsule or the like.

Examples of the vehicle to be used include lactose, corn starch, white sugar, dextrose, sorbitol, crystalline cellulose and silicon dioxide. Examples of the binder include polyvinyl alcohol, polyvinyl ether, ethylcellulose, methylcellulose, gum arabic, tragacanth, gelatin, shellac, hydroxypropylcellulose, hydroxypropylstarch, polyvinylpyrrolidone, white sugar and sorbitol. Examples of the disintegrating agent include starch, agar-agar, gelatin powder, crystalline cellulose, calcium carbonate, sodium bicarbonate, calcium citrate, dextrin and pectin. Examples of the lubricant include magnesium stearate, talc, polyethylene glycol, silica and hardened vegetable oil. The coloring agent is any one which is permitted to be

added to medicines. Examples of the corrigent include cocoa powder, menthol, aromatic powder, peppermint oil, borneol and cinnamon powder. These tablets and granules may be coated with sugar, gelatin or the like.

When a liquid preparation for oral administration is manufactured, a principal agent is, if necessary, mixed with corrigent, buffer, stabilizer or the like, and formed into a syrup or the like according to an ordinary method.

When an injection is manufactured, a principal agent is, if necessary, mixed with pH-regulator, buffer, suspending agent, dissolution auxiliary, stabilizer, isotonizing agent, preservative or the like, and formed into subcutaneous, intramuscular or intravascular injection according to an ordinary method.

Examples of the suspending agent include methylcellulose, polysolvate 80, hydroxyethylcellulose, gum arabic, tragacanth powder, sodium carboxymethylcellulose and polyoxyethylene sorbitan monolaurate. Examples of the dissolution auxiliary include polyoxyethylene-hardened castor oil, polysolvate 80, nicotinamide, polyoxyethylene sorbitan monolaurate, macrogol. Examples of the stabilizer include sodium sulfite, sodium metasulfite and ethers. Examples of the preservative include methyl p-hydroxybenzoate, ethyl p-hydroxybenzoate, sorbic acid, phenol, cresol

and chlorocresol.

Preparative Examples will be described for reference.

Preparative Example 1

Preparation of 3,7,11,15-tetramethyl-2,4,6,10,14-hexadecapentaenoic acid

28.6 g of triethylphosphonoacetate was added to a suspension consisting of 5.0 g of 55 % sodium hydride (oily) and 60 mℓ of n-hexane. The resulting solution was heated under reflux and 20 g of 6,10,14-trimethylpentadeca-3,5,9,13-tetraen-2-one was added dropwise to the solution under stirring. After 30 minutes, the reaction mixture was poured onto 200 mℓ of ice-water and extracted with 500 mℓ of hexane. The hexane layer was twice washed with 100 mℓ of a mixture of methanol and water (2=1) and concentrated. The concentrate was purified by silica gel column chromatography to obtain 18 g of ethyl 3,7,11,15-tetramethyl-2,4,6,10,14-hexadecapentaenoate.

3.9 g of potassium hydroxide was dissolved in 30 mℓ of isopropyl alcohol. 10 g of the above hexadecapentaenoate ester was added to the resulting solution. The mixture was stirred at 50°C for one hour. The reaction mixture was poured onto ice-water, made acid with hydrochloric acid and extracted with

100 mℓ of ethyl ether. The ether layer was washed with water, dried over magnesium sulfate and concentrated to obtain 9.0 g of an oil. The oil was dissolved in 50 mℓ of n-hexane and crystallized at -20°C to obtain 4.0 g of 3,7,11,15-tetramethyl-2,4,6,10,14-hexadecapentaenoic acid as a pale yellow needle crystal.

melting point: 78.4°C

mass spectrum (m/e): 302 ($M^+$)

infrared absorption spectrum ($cm^{-1}$, KBr tablet):

3450, 2900, 1680, 1595

NMR spectrum (δ, $CDCl_3$): 1.61 (6H,s), 1.68 (3H,s), 1.86 (3H,s), 1.92 to 2.24 (8H,b), 2.35 (3H,s), 5.10 (2H,b), 5.76 (1H, bs), 5.98 (1H,d,J=11Hz), 6.20 (1H,d,J=15Hz), 6.90 (1H,dd,J=11Hz, 15Hz), 11.63 (1H,b)

ultraviolet absorption spectrum: $\lambda_{max}^{methanol}$ 304 nm.

Claims

1. A pharmaceutical use of 3,7,11,15-tetramethyl-2,4,6,10,14-hexadecapentaenoic acid or a salt thereof for enhancing the anti-tumor effect.

2. A pharmaceutical use of 3,7,11,15-tetramethyl-2,4,6,10,14-hexadecapentaenoic acid or a salt thereof for preparing a pharmaceutical composition to enhance the anti-tumor effect.

3. A pharmaceutical composition which comprises (A) 3,7,11,15-tetramethyl-2,4,6,10,14-hexadecapentaenoic acid or a salt thereof and (B) an anti-tumor agent.